# EUROPEAN PATENT APPLICATION

(11) **EP 1 582 160 A1**
(43) Date of publication of application: **05.10.2005**
(21) Application number: 04007786.9
(22) Date of filing: 31.03.2004
(51) Int. Cl.: A61B 17/72

(54) **Intramedullary nail comprising elements of shape-memory material**

(71) Applicant: ORTHOFIX INTERNATIONAL B.V., 1071 Amsterdam (NL)
(72) Inventor: Coati, Michele, 37029 San Pietro in Cariano (Verona) (IT); Marazzi, Giancarlo, 21047 Saronno (Varese) (IT); Marini, Graziano, 37060 Castel D'Azzano (Verona) (IT); Rossi, Graziano, 37139 Verona (IT); Rossi, Luigi, 37019 Peschiera del Garda (Verona) (IT); Venturini, Daniele, 37064 Povegliano Veronese (Verona) (IT)
(74) Representative: Ferreccio, Rinaldo

(57) **Abstract**

An intramedullary nail (10, 110, 210, 310) suitable for insertion in a fractured elongate bone (12, 112, 212, 312) and for an unusually simple fixation therein comprises a substantially straight stem (14, 114, 214, 314) extending between a proximal end (16, 116, 216, 316) and a distal end (18, 118, 218, 318), said substantially straight stem (14, 114, 214, 314) being equipped with a plurality of elements (20, 120, 220, 320) realised with a shape-memory material, a free end (21, 121, 221, 321) of said elements (20, 120, 220, 320) being positioned outwards the stem (14, 114, 214, 314) for the nail fixation to the bone.

## Description

### Field of application

The present invention relates in its more general aspect to an intramedullary nail suitable for insertion in a fractured elongate bone and an application method of said nail in said bone.

In particular, the invention relates to an intramedullary nail suitable for insertion in a fractured elongate bone, such as a femur or a tibia, comprising a substantially straight stem extending between a proximal end and a distal end.

### Prior art

Intramedullary nails are known, which, during a surgical operation, are inserted in a fractured elongate bone and fixed therein, in order to reconstruct the original bone shape and in the meantime to recover the bone solidity, so that callus regeneration mechanisms can correctly occur.

The stems of these intramedullary nails are generally cylinder-shaped and they can be both solid and hollow.

In order to fix the intramedullary nail to the bone portions to be reconstructed, two offset holes are usually provided on the nail, having axes lying on parallel planes and extending diametrically across the stem, in correspondence with the nail distal end, and two offset holes having the same size, having axes not necessarily lying on parallel planes, in correspondence with the nail proximal end. Said holes are suitable for housing bone screws, which are inserted, after a convenient bone drilling, in the bone, with the subsequent fixation of the intramedullary nail to the bone portions.

Although advantageous under different points of view, intramedullary nails being structured as above schematically described have known drawbacks mainly occurring when bone drillings are to be performed for bone screw insertion. This step is particularly critical since it is known that a good nail fastening essentially depends on the correct realisation of these bone drillings, obviously made in correspondence with the holes of the inserted intramedullary nail.

However the precise location of the intramedullary nail holes is made difficult by the fact that the holes are no more visible, being the nail inserted in the bone. It is then worth underlining a further location problem, i.e the fact that the intramedullary nail can be, when being inserted, slightly bent, so that the holes at the nail distal end are no more, with respect to the proximal end, in the same position as before installing the nail.

The traditional technique for locating the holes of an inserted intramedullary nail provides the use of X rays, involving however the well known risks of cumulative exposure of the operating staff, besides being quite awkward during the surgical operation.

Specific mechanical devices have thus been studied and realised, such as for example the one described in the European patent no. EP 772 420 in the name of the same Applicant.

These devices do not require the use of X rays, but they have the drawback of requiring several operational steps, all to be performed quite carefully and precisely.

### Summary of the invention

The problem underlying the present invention is to provide an intramedullary nail suitable for insertion in a fractured elongate bone, capable to meet the above-mentioned requirement, meanwhile overcoming, in a simple and effective way, all the drawbacks mentioned with reference to the prior art.

This problem is solved, according to the present invention, by an intramedullary nail suitable for insertion in a fractured elongate bone, as above described and characterised in that said substantially straight stem comprises a plurality of elements realised with at least a shape-memory material, a free end of said elements being positioned outwards the stem for the nail fixation to the bone.

Further features and the advantages of the intramedullary nail suitable for insertion in a fractured elongate bone, as well as of the application method of said nail in said fractured bone, according to the present invention, will be apparent from the following description of an embodiment thereof made with reference to the attached drawings, given by way of non-limiting example.

### Brief description of the drawings

Figure 1 is a schematic perspective view of a first embodiment of an intramedullary nail, suitable for insertion in a fractured elongate bone, according to the present invention.
Figure 2 is a schematic perspective view of the nail of figure 1, in a different shape.
Figure 3 is a schematic perspective enlarged view of the nail of figure 1.
Figure 4 is a schematic perspective enlarged view of the nail of figure 2.
Figure 5 is a schematic perspective view of a second embodiment of an intramedullary nail, suitable for insertion in a fractured elongate bone, according to the present invention.
Figure 6 is a schematic perspective view of the nail of figure 1, in a different shape.
Figure 7 is a schematic perspective enlarged view of the nail of figure 5.
Figure 8 is a schematic perspective enlarged view of the nail of figure 6.
Figure 9 is a schematic perspective partial view of a third embodiment of an intramedullary nail, suitable for insertion in a fractured elongate bone, according to the present invention.
Figure 10 is a schematic perspective partial view of a forth embodiment of an intramedullary nail according to the present invention.

### Detailed description

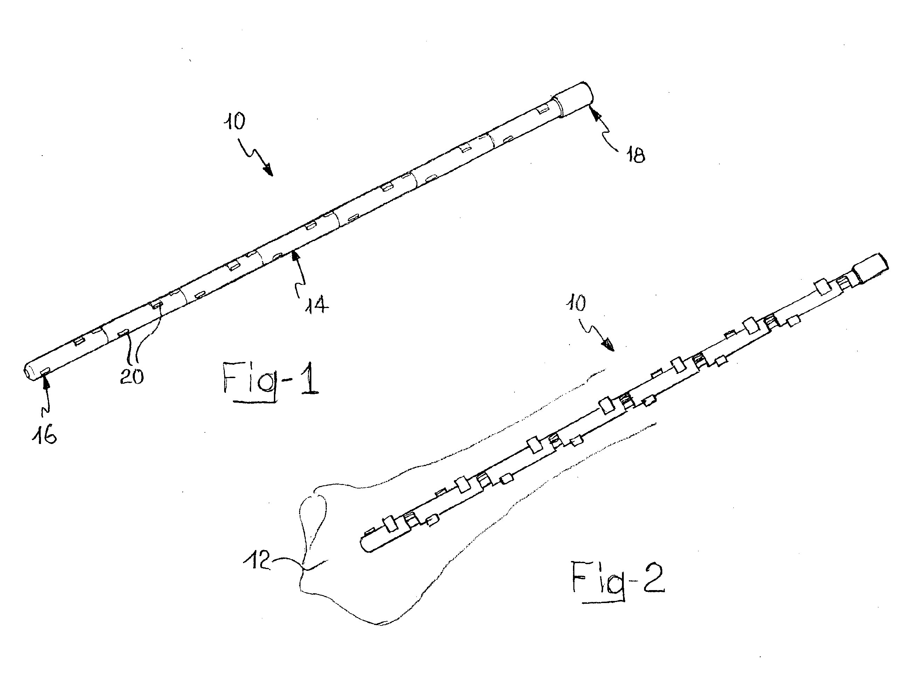

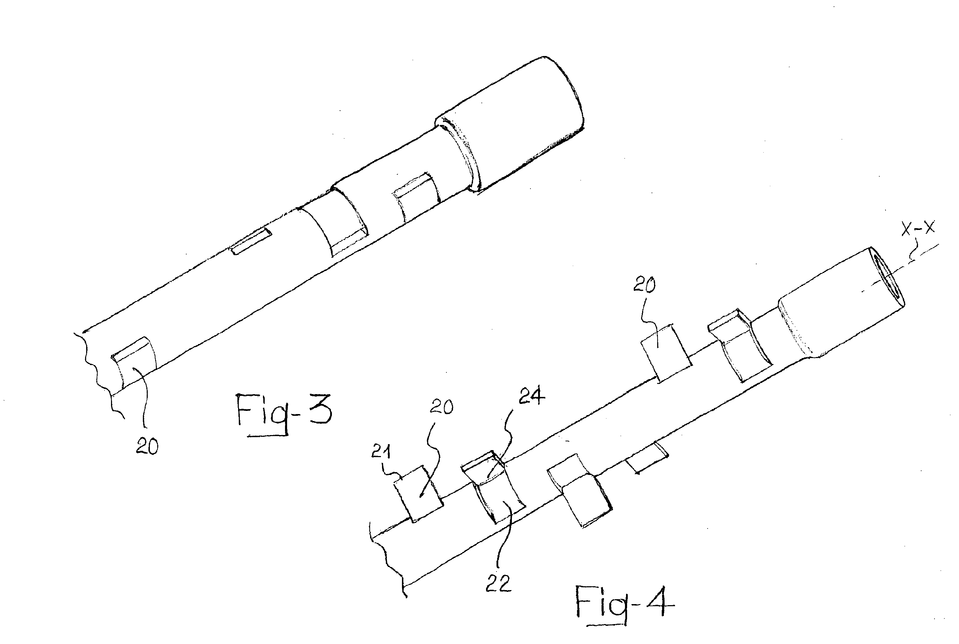

Referring first to figures 1, 2, 3 and 4, an intramedullary nail according to the present invention is shown and globally indicated with 10, suitable for insertion in a fractured elongate bone 12, such as for example a femur or a tibia.

The nail 10 comprises a substantially straight stem 14 extending between a proximal end 16 and a distal end 18.

The stem 14 is preferably composed of a cylindrical tubular body. The stem 14, according to an aspect of the present invention, comprises a plurality of elements 20 realised with at least a shape-memory material, a free end 21 of said elements 20 being positioned outwards the stem 14 in a final shape corresponding to the nail 10 fixation to said fractured bone 12.

Shape memory material means a material having a given initial starting shape and taking, under predetermined external conditions (for example a temperature rise and/or drop) or undergoing a predetermined activation condition, i.e. after a so-called "material instruction" step, a given new shape.

Known shape-memory materials which are suitable for use in the present invention are, for instance, certain nickel-titanium alloys.

The invention is based on the following principle: subjecting an element realised in a shape memory material, having a predetermined initial shape at rest, to said so-called "material instruction" step (for example to a predetermined temperature variation), said element takes a shape being maintained as long as the "instruction" step effect persists; said shape, being different from the initial shape, can be called transient shape and it is temporary or unstable. When the "instruction" step effect stops, the element leaves said transient shape and, coming back towards the initial shape, takes a working shape. Depending on the type of material and on the working temperature, in the working shape, said material can arrive at the initial shape or it can go further on the initial shape, arriving at a final shape. It is worth pointing out that the more this final shape is far from the initial shape, the more the available shape memory energy of the material is high.

The invention has been reached as result of the intuition that an element realised in a shape memory material, in the passage from the transient shape towards the working shape, can create the fixing of the intramedullary nail in the bone.

Preferably, the plurality of elements 20 are distributed in a substantially uniform way on the side surface of the stem 14.

In particular, in the example of figure 1, each element 20 is housed in a corresponding groove 22 circumferentially developing along the side surface of the stem 14, said groove 22 generally having a U-shaped profile.

The element 20 has the shape of a cylindrical crown sector and the thickness thereof is substantially equal to the depth of the groove 22. This element 20 is fixed, for example by laser welding or keying, along a side end 24 thereof (substantially developing along the axis of the stem 14) in the groove 22.

The above description corresponds to the initial shape of the element 20. In the final shape, leading to the fixation of the nail 10 to said fractured bone 12, the free end 21 of the element 20, being the opposite end to said side end 24, is positioned outwards the stem 14: for example, the sector of a cylindrical crown of the initial shape straightens, substantially becoming a parallelepiped.

Preferably, in the series of grooves 22, the elements 20, all having the same angular development, are offset to each other. In the example of the figures, three further elements arranged in different angular positions exist between two elements 20 arranged in the same angular position.

Moreover, always preferably, the side ends 24 wherein each element 20 is fixed are, in the series of grooves 22, alternate to each other i.e., observing the stem 14 in the vertical position, at one time the side end 14 is on the left and next time it is on the right.

The stem 14 can be connected with an outer template by means of a side thread.

It must be observed that the circumferentially-developing grooves 22 can be realised along the whole circumference or they can be developed only along the circumference arc corresponding to the cylindrical crown sector of the elements 20.

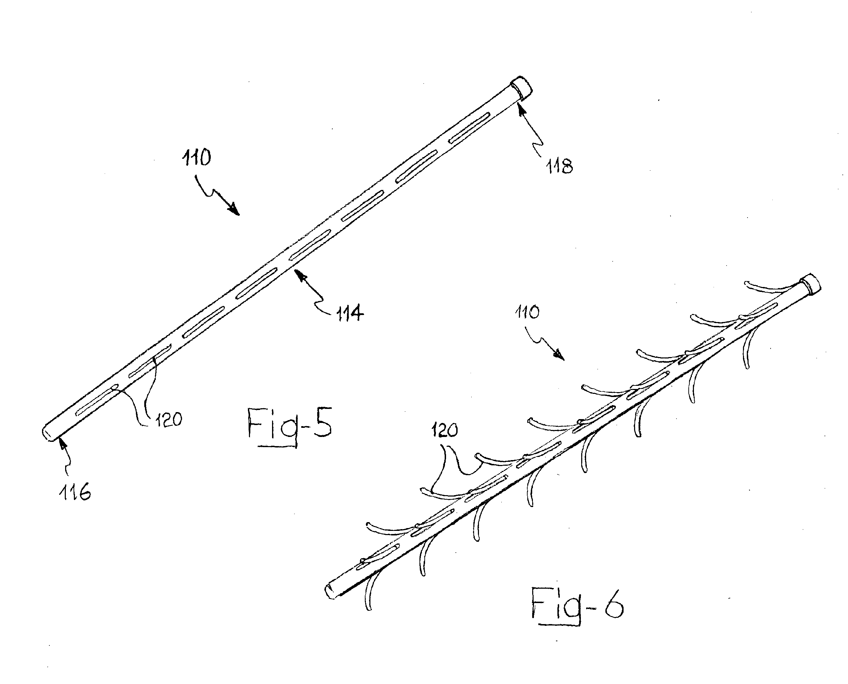

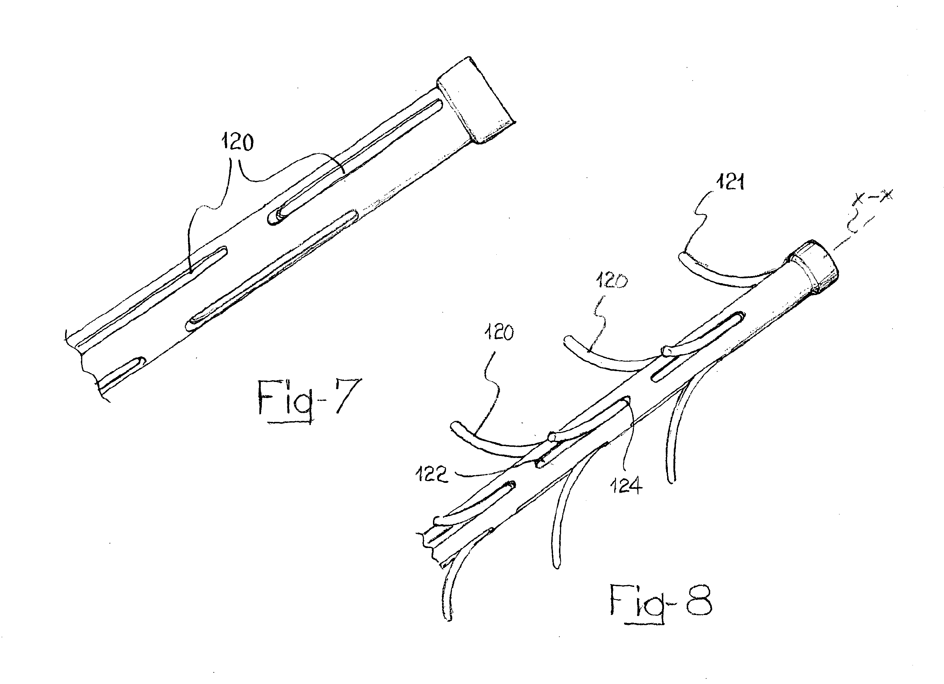

Referring now to figures 5, 6, 7 and 8, a second embodiment of the intramedullary nail, according to the present invention is shown and globally indicated with 110, suitable for insertion in a fractured elongate bone 112. In this embodiment, elements being structurally or functionally similar to the elements of the intramedullary nail 10 are indicated with the same reference number increased by 100 and the detailed description thereof is not repeated hereafter.

The nail 110 comprises a substantially straight stem 114 extending between a proximal end 116 and a distal end 118.

The stem 114 is preferably composed of a cylindrical tubular body. The stem 114, according to an aspect of the present invention, comprises a plurality of elements 120 realised with at least a shape-memory material, a free end 121 of said elements 120 being positioned outwards the stem 114 in a final shape corresponding to a fixation of the nail 110 to said fractured bone 112.

Preferably, the plurality of elements 120 are distributed in a substantially uniform way on the side surface of the stem 114.

In particular, in the example of figure 5, each element 120 is housed in a corresponding groove 122 axially developing along the side surface of the stem 114, said groove 122 generally having a U-shaped profile.

The element 120 is cylinder-shaped and the thickness thereof is substantially equal to the depth of the groove 122. This element 120 is fixed in the groove 122, (for example by laser welding) in correspondence with the front end 124 thereof.

The above description corresponds to the initial shape of the element 120. In the final shape, leading to the fixation of the nail 110 to said fractured bone 112, the free end 121 of the element 120, being the opposite end to said front end 124, is positioned outwards the stem 114: for example, the straight-developing cylinder of the initial shape bends outwards the stem 114, taking thus a curvilinear development.

Preferably, in the series of grooves 122, the elements 120, all having the same length, are arranged with the front ends 124 being offset to each other. In the example of the figures, two further elements arranged with the front ends 124 in different angular positions exist between the front ends 124 of two elements 120 arranged in the same angular position.

Moreover, always preferably, the front ends 124 wherein the elements 120 are fixed are all those turned towards a same end (proximal 116 or distal 118) of the nail 110: this serves to facilitate a possible extraction of the nail 110.

The stem 114 can be connected with an outer template by means of a side thread.

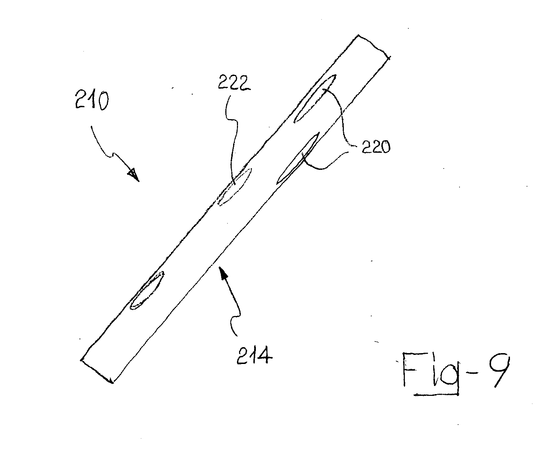

Referring now to figure 9, a third embodiment of the intramedullary nail, according to the present invention is shown and globally indicated with 210, suitable for insertion in a fractured elongate bone 212. In this embodiment, elements being structurally or functionally similar to the elements of the intramedullary nail 10 are indicated with the same reference number increased by 200 and the detailed description thereof is not repeated hereafter.

The nail 210 comprises a substantially straight stem 214 extending between a proximal end 216 and a distal end 218.

The stem 214 is preferably composed of a cylindrical tubular body. The stem 214, according to an aspect of the present invention, comprises a plurality of elements 220 realised with at least a shape-memory material, a free end 221 of said elements 220 being positioned outwards the stem 214 in a final shape corresponding to a fixation of the nail 210 to said fractured bone 212.

Preferably, the plurality of elements 220 are distributed in a substantially uniform way on the side surface of the stem 214.

In particular, in the example of figure 9, each element 220 is housed in a corresponding groove 222 axially developing along the side surface of the stem 214.

The thickness of the element 220 is substantially equal to the depth of the groove 222, said element 220 being fixed in the groove 222, (for example by laser welding) in correspondence with a bottom 224 thereof.

The above description corresponds to the initial shape of the element 220. In the final shape, leading to the fixation of the nail 210 to said fractured bone 212, the free end 221 of the element 220, being the opposite end to said bottom 224, is positioned outwards the stem 114.

For example, the element 220 is a shape-memory spring, which is a known on sale component, which, in the final shape, bends outwards the stem 214.

Preferably, in the series of grooves 222, the elements 220, all having the same length, are offset to each other.

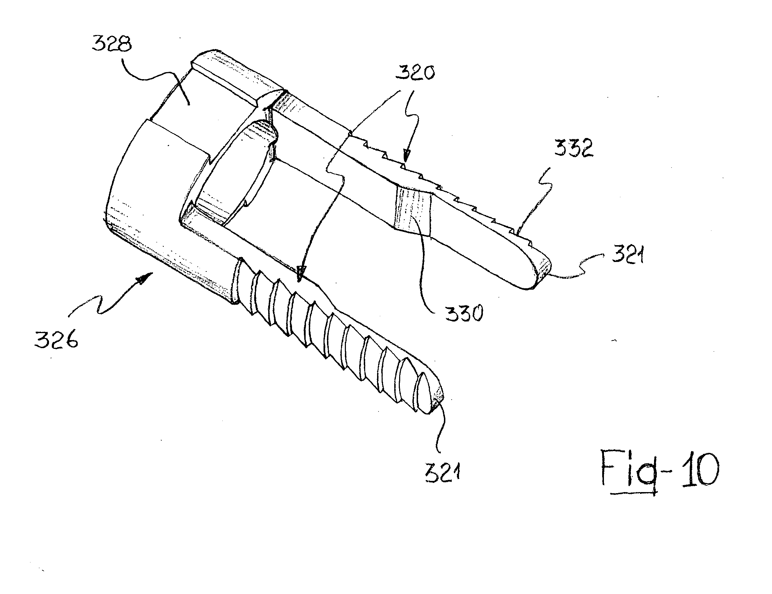

Referring now to figure 10, a portion of a forth embodiment of the intramedullary nail, according to the present invention, is shown and globally indicated with 310, suitable for insertion in a fractured elongate bone 312. In this embodiment, elements being structurally or functionally similar to the elements of the intramedullary nail 10 are indicated with the same reference number increased by 300 and the detailed description thereof is not repeated hereafter.

The nail 310 comprises a substantially straight stem 314 extending between a proximal end 316 and a distal end 318.

The stem 314 is preferably composed of a cylindrical tubular body. The stem 314, according to an aspect of the present invention, comprises a plurality of elements 320 realised with at least a shape-memory material, a free end 321 of said elements 320 being positioned outwards the stem 314 in a final shape corresponding to a fixation of the nail 310 to said fractured bone 312.

Preferably, the plurality of elements 320 are distributed in a substantially uniform way on the side surface of the stem 314. Always preferably, the elements 320 are offset to each other, for example they are arranged with an offset of 90 sexagesimal degrees.

In particular, in the example of figure 10, said elements 320 are mounted on cylindrical sleeves 326. More precisely, a plurality of said cylindrical sleeves 326 are worn, close to each other in series, on said stem 314, each of said cylindrical sleeves 326 being equipped at one end with two elements 320. Said two elements 320 are two opposite fillets substantially positioned with the axis of said sleeve 326.

Two opposite grooves 328 are also provided on said cylindrical sleeve 326, substantially offset by a right angle with respect to said two fillets and suitable for housing at least partially the fillets of the adjacent cylindrical sleeve 326: therefore the sleeves 326 are partially overlapped to each other.

The free ends 321 of said fillets is positioned outwards the stem 314 for fixing the nail 310 to the bone: in other words, the fillets have a flexural memory.

The fillet profile is tapered towards the free end 321. Preferably, on the fillet surface turned towards the stem 314, a section is provided, which has a sloping surface 330 leading to a tapering of the fillet thickness in correspondence with the section towards the free end 321.

The fillets, on the surface turned outwards the stem 314, have a substantially saw-toothed profile 332 to increase the fillet grip on the bone.

An application method, according to the present invention, of said intramedullary nail in said elongate bone comprises:
a location step of said nail in said elongate bone, to mend the fracture;
an activation step of said plurality of elements realised with at least a shape-memory material, taking said final form corresponding to a fixation of the nail to said fractured bone.

The operation of the intramedullary nail, suitable for insertion in a fractured elongate bone, according to the present invention, is already partially evident from the description of the above application method and it is specified hereafter.

In the case of the first embodiment of the intramedullary nail 10, the nail 10 is positioned in said elongate bone 12, in the initial shape thereof, with the free ends 21 of the elements 20 substantially not projecting from the side surface of the stem 14.

Afterwards, in order to fix the nail 10 to said bone 12, said shape-memory elements 20 are activated, so that the free ends 21 tend to be positioned outwards the stem 14, with a subsequent interference of these free ends 21 with respect to the bone portion surrounding said stem 14: this interference causes in practise a grip, solidly fixing said nail 10 in the bone 12.

It must be noticed how advantageously the grooves 22 are offset along the stem 14 to affect in a limited way the cutting effect.

Moreover, the side ends 24 wherein each element 20 is fixed are alternate to each other not to create a torsion or rotation effect when stabilising the nail 10 on the bone 12.

It is also worth underlining that the stem 14 is preferably a cylindrical tubular body: the central hole serves to have an improved thermal exchange, helping thus in activating the elements 20.

In the case of the second embodiment of the intramedullary nail 110, the nail 110 is positioned in said elongate bone 112, in the initial shape thereof, with the free ends 121 of the elements 120 substantially not projecting from the side surface of the stem 114.

Afterwards, in order to fix the nail 110 to said bone 112, said shape-memory elements 120 are activated, so that the free ends 121 tend to be positioned outwards the stem 114, with a subsequent interference of these free ends 121 with respect to the bone portion surrounding said stem 114: this interference causes in practise a grip, solidly fixing said nail 110 in the bone 112.

It must be noticed how advantageously shape-memory wires, being known and easily available on the market, are used as elements 120, the material instruction step of these wires being also particularly easy.

Moreover, the wire arrangement with the axis of the stem 114 allows the wire free ends 121 to be positioned, in the final shape, far apart from the side surface of the stem 114.

Furthermore, the curvilinear development taken by the wire in the final shape prevents it from collapsing in the bone 112, favouring thus a possible extraction thereof. The extraction is also favoured by the use of shape-memory wires, which, during the extraction, are positioned in the grooves 122.

In the case of the third embodiment of the intramedullary nail 210, the nail 210 is positioned in said elongate bone 212, in the initial shape thereof, with the free ends 221 of the elements 220 substantially not projecting from the side surface of the stem 214.

Afterwards, in order to fix the nail 210 to said bone 212, said shape-memory elements 220 are activated, so that the free ends 221 tend to be positioned outwards the stem 214, with a subsequent interference of these free ends 221 with respect to the bone portion surrounding said stem 214: this interference causes in practise a grip, solidly fixing said nail 210 in the bone 212.

In the case of the forth embodiment of the intramedullary nail 310, the nail 310 is positioned in said elongate bone 312, in the initial shape thereof, with the free ends 321 of the elements 320 substantially not projecting from the side surface of the stem 314. The stem 314 serves as structure.

Afterwards, in order to fix the nail 310 to said bone 312, said shape-memory elements 320 are activated, so that the free ends 321 tend to be positioned outwards the stem 314, with a subsequent interference of these free ends 321 with respect to the bone portion surrounding said stem 314: this interference causes in practise a grip, solidly fixing said nail 310 in the bone 312.

The tapering of the fillet thickness allows a variable rigidity in the fillet to be obtained: therefore a fillet allowing high loads for adjacent diametral measures and lower loads for high diametral measures is obtained.

It is underlined that, during a possible extraction of the nail according to the invention, it is convenient to insert in the hole of the stem tubular body an insert reducing the nail temperature in order to cause a phase shift in the material structure, thus obtaining a lower bending resistance of the shape-memory elements.

The main advantage achieved by the intramedullary nail suitable for insertion in a fractured elongate bone, as well as by the application method of said nail in said bone, according to the present invention, is the fact of unusually simplifying the fixation step of the intramedullary nail inserted in the bone. In practise, the nail fixation is obtained simultaneously with the insertion thereof in the bone, since the shape-memory elements, cooled at first to take the initial shape, take again in the bone their final shape, due to the heat released by the patient's body.

Another advantage of the intramedullary nail suitable for insertion in a fractured elongate bone, according to the present invention, is to prevent undesired nail torsions or rotations.

Obviously, in order to meet specific and contingent requirements, a skilled in the art could bring several changes to the above-described intramedullary nail suitable for insertion in a fractured elongate bone and application method of said nail in said bone, all however comprised in the scope of protection of the present invention, as defined in the following claims.

## Claims

1. Intramedullary nail (10, 110, 210, 310) suitable for insertion in a fractured elongate bone (12, 112, 212, 312), comprising a substantially straight stem (14, 114, 214, 314) extending between a proximal end (16, 116, 216, 316) and a distal end (18, 118, 218, 318), **characterised in that** said substantially straight stem (14, 114, 214, 314) comprises a plurality of elements (20, 120, 220, 320) realised with at least a shape-memory material, a free end (21, 121, 221, 321) of said elements (20, 120, 220, 320) being positioned outwards the stem (14, 114, 214, 314) for the nail fixation to the bone.

2. Intramedullary nail (10, 110, 210, 310) according to claim 1, **characterised in that** said plurality of elements (20, 120, 220, 320) are distributed in a substantially uniform way on the side surface of the stem (14, 114, 214, 314).

3. Intramedullary nail (10) according to claim 1, **characterised in that** each element (20) is housed in a corresponding groove (22) circumferentially developing along the side surface of the stem (14).

4. Intramedullary nail (110, 210) according to claim 1, **characterised in that** each element (120, 220) is housed in a corresponding groove (122, 222) axially developing along the side surface of the stem (114, 214).

5. Intramedullary nail (10, 110) according to claim 3 or 4, **characterised in that** said groove (22, 122) has a U-shaped profile.

6. Intramedullary nail (10) according to claim 3, **characterised in that** each element (20) has the shape of a cylindrical crown sector and the thickness thereof is substantially equal to the depth of the groove (22), said cylindrical crown sector straightening for the nail fixation to the bone.

7. Intramedullary nail (10) according to claim 6, **characterised in that** said element (20) is fixed along a side end (24) thereof, substantially developing along the axis of the stem (14) in the groove (22).

8. Intramedullary nail (10) according to claim 7, **characterised in that** said element (20) is fixed by laser welding or keying.

9. Intramedullary nail (10, 110, 210, 310) according to claim 1, **characterised in that** said elements (20, 120, 220, 320) are offset to each other.

10. Intramedullary nail (10) according to claim 7, **characterised in that** the side ends (24) wherein each element (20) is fixed are, in the series of grooves (22), alternate to each other.

11. Intramedullary nail (10, 110, 210, 310) according to claim 1, **characterised in that** said stem (14, 114, 214, 314) is composed by a cylindrical tubular body.

12. Intramedullary nail (10, 110, 210, 310) according to claim 1, **characterised in that** said stem (14, 114, 214, 314) is connected to an outer template by means of a side thread.

13. Intramedullary nail (110) according to claim 3, **characterised in that** each element (120) is cylinder-shaped and the thickness thereof is substantially equal to the depth of the groove (122), said cylindrical shape bending outwards the stem (114) for the nail fixation to the bone.

14. Intramedullary nail (110) according to claim 13, **characterised in that** said element (120) is fixed in the groove (122) in correspondence with a front end thereof (124).

15. Intramedullary nail (110) according to claim 14, **characterised in that** said element (120) is fixed by laser welding.

16. Intramedullary nail (110) according to claim 14, **characterised in that** said front ends (124) wherein the elements (120) are fixed are all those turned towards a same proximal (116) or distal (118) end of the nail (110).

17. Intramedullary nail (210) according to claim 4, **characterised in that** the thickness of each element (220) is substantially equal to the depth of the groove (222).

18. Intramedullary nail (210) according to claim 17, **characterised in that** said element (220) is fixed in the groove (222) in correspondence with a bottom thereof (224).

19. Intramedullary nail (210) according to claim 18, **characterised in that** said element (220) is fixed by laser welding.

20. Intramedullary nail (210) according to claim 4 or 17,
**characterised in that** each element (220) is a shape-memory spring, bending outwards the stem (214) for the nail fixation to the bone.

21. Intramedullary nail (310) according to claim 1, **characterised in that** said elements (320) are mounted on cylindrical sleeves (326), a plurality of said cylindrical sleeves (326) being put, close to each other in series, on said stem (314), each of said cylindrical sleeves (326) being equipped at one end with two of said elements (320).

22. Intramedullary nail (310) according to claim 21, **characterised in that** said two elements (320) are opposite fillets substantially positioned with the axis of said sleeve (326), two opposite grooves (328) being provide on said cylindrical sleeve (326), substantially offset by a right angle with respect to said two fillets and suitable for housing at least partially the fillets of the adjacent cylindrical sleeve (326).

23. Intramedullary nail (310) according to claim 22, **characterised in that** the fillet profile is tapered towards the free end (321).

24. Intramedullary nail (310) according to claim 23, **characterised in that**, on the fillet surface turned towards the stem (314), a section is provided, which has a sloping surface (330) leading to a tapering of the fillet thickness in correspondence with the section towards the free end (321).

25. Intramedullary nail (310) according to claim 22, **characterised in that** the fillets, on the surface turned outwards the stem (314), have a substantially saw-toothed profile (332).

26. An application method, in a fractured elongate bone (12, 112, 212, 312), of an intramedullary nail (10, 110, 210, 310) according to claim 1, **characterised in that** it comprises:
a location step of said nail (10, 110, 210, 310) in said elongate bone (12, 112, 212, 312), to mend the fracture;
an activation step of said plurality of elements (20, 120, 220, 320) realised with at least a shape-memory material, a free end (21, 121, 221, 321) of said elements (20, 120, 220, 320) being positioned outwards the stem (14, 114, 214, 314) for the nail fixation to the bone.
